# DEMANDE DE BREVET EUROPEEN

(11) **EP 2 425 767 A1**
(43) Date de publication de la demande: **07.03.2012**
(21) Numéro de dépôt: 11169125.9
(22) Date de dépôt: 08.06.2011
(51) Int. Cl.: A61B 5/00, G01K 13/00, A61B 5/01

(54) **Système de mesure de la tempérture interne d'un sujet.**

(30) Priorité: 02.09.2010 FR 1056964
(71) Demandeur: Binet, Marc-Hervé, 74110 Avoriaz (FR)
(72) Inventeur: Binet, Marc-Hervé, 74110 Avoriaz (FR)
(74) Mandataire: Casalonga, Axel

(57) **Abrégé**

Le système de mesure de la température interne d'un sujet, comprend un capteur (5) de mesure de température et un dispositif d'affichage (7) apte à afficher la température déterminée.

Le système comprend en outre :
une cartographie (11) comprenant un ensemble de valeurs de température associée chacune à des informations relatives à une ou plusieurs actions à entreprendre, et
un moyen de détermination (12) d'au moins une action à entreprendre en fonction des informations contenues dans la cartographie (11) et de la température mesurée.

## Description

L'invention a pour domaine technique les systèmes de mesure de température, et plus particulièrement, les systèmes de mesure de la température d'un être vivant.

Il est connu de mesurer la température d'un être vivant, notamment d'un mammifère, au niveau des zones comprenant une grande densité de vaisseaux sanguins. Il est ainsi de possible d'obtenir la température interne d'un être vivant par mesure de la température externe au niveau de la bouche, de la tête, de l'aine, de l'aisselle, ou du rectum.

Les dispositifs de mesure de température comprennent généralement un transducteur sensible aux variations de température de façon que l'évolution d'un repère visuel puisse être corrélée à une variation de température entre la température de repos du transducteur et la température externe de l'être vivant. Il est alors possible de déterminer la température interne de l'être vivant en fonction de la température externe obtenue et de la partie du corps de l'être vivant à partir de laquelle la température externe a été obtenue.

Par extension, il est possible d'utiliser comme transducteur un capteur sensible au rayonnement infrarouge afin de déterminer la température externe d'un être vivant, et d'utiliser un affichage électronique, par exemple un affichage LCD, comme repère visuel. En effet, la chaleur à l'origine d'une température se dissipe sous forme de rayonnement infrarouge. La thermodynamique et l'électromagnétisme prévoient que l'énergie thermique et le rayonnement infrarouge sont directement liés et que l'intensité du rayonnement infrarouge diminue en fonction de la distance et du milieu parcourus. En prenant ces différents paramètres en compte, il est ainsi possible de déterminer la température interne d'un être vivant en déterminant l'intensité du rayonnement infrarouge émis.

Ces principes ont été concrétisés dans les dispositifs de mesure de température à distance et dans les caméras infrarouges. Les dispositifs de mesure de température à distance, également appelés pyromètres, ont été adaptés aux domaines médical et vétérinaire. De tels thermomètres infrarouges permettent la prise de température d'un être vivant à distance et sans contact, diminuant ainsi les risques de contaminations croisées ou de stress induit par la prise de température.

Toutefois, l'interprétation de la température mesurée reste le domaine du professionnel de santé, et ce en dépit de la vulgarisation de tels dispositifs.

Il existe ainsi un besoin pour un dispositif de mesure de la température qui soit apte à conseiller l'utilisateur sur l'attitude à tenir en fonction de la température mesurée.

Il existe également un besoin pour un dispositif de mesure de la température utilisable par un individu autre qu'un professionnel de santé qui puisse rendre disponible un historique des prises de températures.

Selon un mode de réalisation, on définit un système de mesure de la température interne d'un sujet, comprenant un capteur de mesure de température et un dispositif d'affichage apte à afficher la température mesurée. Le système de mesure comprend en outre :
une cartographie comprenant un ensemble de valeurs de température associée chacune à des informations relatives à une ou plusieurs actions à entreprendre,
un moyen de détermination d'au moins une action à entreprendre en fonction des informations contenues dans la cartographie et de la température mesurée, et
au moins une mémoire (13) apte à mémoriser des paramètres des actions entreprises en rapport avec le sujet dont la température est mesurée.

Le système de mesure peut comprendre au moins une mémoire, apte à mémoriser des paramètres physiques d'au moins un sujet dont la température est mesurée.

Les paramètres physiques peuvent être choisis parmi la liste comprenant l'espèce, l'âge, le poids et le sexe du sujet.

Le système de mesure peut comprendre au moins une mémoire, apte à mémoriser des paramètres physiques de l'environnement du sujet dont la température est mesurée.

Les paramètres physiques de l'environnement peuvent être choisis parmi la température de l'air, l'hygrométrie, la date courante, l'heure courante et le rythme circadien.

Les paramètres des actions entreprises peuvent être choisis parmi la date et l'heure d'au moins la dernière action entreprise.

Le capteur de mesure de température peut être de type sans contact à distance. La mesure de température sans contact à distance sera réalisée notamment dans les zones frontales et temporales.

Le capteur de mesure de température peut être de type à contact.

Le capteur de mesure de température peut être apte à déterminer la température d'un sujet par mesure au niveau du conduit auditif externe.

Le capteur de mesure de température peut être apte à déterminer la température d'un sujet par mesure en contact au niveau de l'orifice buccal.

Le dispositif d'affichage peut être déporté et relié par un moyen de communication à distance.

Le dispositif d'affichage peut être apte à afficher simultanément la température du sujet, au moins une action à entreprendre et au moins un des paramètres choisi parmi les paramètres physiques de l'environnement, les paramètres physiques d'au moins un sujet et/ou les paramètres de prises d'un traitement.

Le dispositif d'affichage, la cartographie et le moyen de détermination peuvent être déportés de façon logicielle sur un dispositif portable.

Un dispositif peut comprendre un dispositif portable apte à échanger des données avec le système de mesure, et à déterminer des conseils et prescriptions en fonction des données échangées avec le système de mesure.

D'autres buts, caractéristiques et avantages de l'invention apparaîtront à la lecture de la description suivante, donnée uniquement à titre d'exemple non limitatif et faite en référence aux dessins annexés sur lesquels :
- la figure 1 représente une vue de coté du dispositif de mesure de la température,
- la figure 2 représente une vue de dessus du dispositif de mesure de la température, et
- la figure 3 représente une vue de l'affichage du dispositif de mesure de la température.

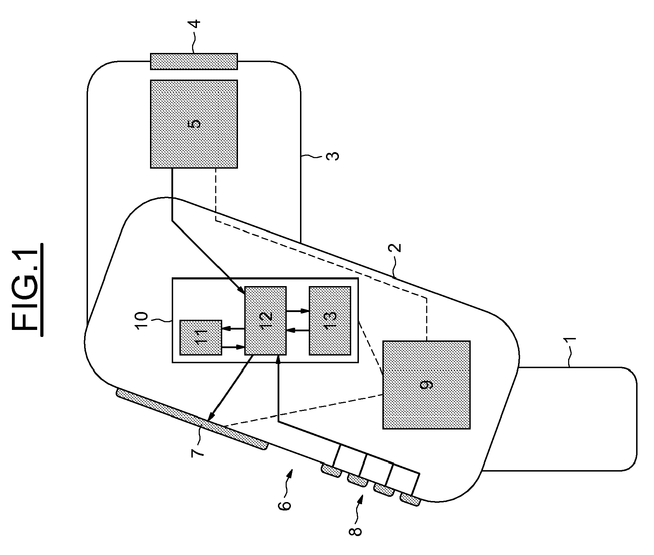

Sur la figure 1, on peut voir un système de mesure de la température comprenant une poignée 1, un corps 2, et un logement 3 comprenant un capteur de température. Le capteur de température 5 peut être un capteur de type avec ou sans contact. Il est ici, par exemple, caractérisé par un capteur apte à déterminer la température du sujet en fonction du rayonnement infrarouge émis.

La poignée 1, le corps 2 et le logement 3 du capteur peuvent être fixés solidairement ou de façon articulée, par exemple de sorte que la poignée 1 et le logement 3 du capteur puissent se replier dans le corps 2.

Le logement 3 du capteur comprend une ouverture 4 apte à laisser passer le rayonnement infrarouge. Cette ouverture 4 peut être réalisée en retirant une partie de l'habillage extérieur du logement 3 du capteur de sorte que le rayonnement infrarouge rentre en contact direct avec le capteur 5. L'ouverture 4 est réalisée avec un matériau transparent dans la gamme de fréquence des infrarouges, c'est-à-dire un matériau présentant peu ou pas d'absorption du rayonnement infrarouge. On peut ainsi concevoir un dispositif fermé isolant le capteur 5 de l'environnement et des dégradations qui pourraient en découler. De préférence, l'ouverture 4 est située dans l'alignement de la poignée 1 et du corps 2 du dispositif de mesure, de sorte que l'utilisateur puisse voir simultanément le sujet dont la température est prise et l'affichage 7 du dispositif de mesure.

Le capteur 5 peut être adapté à une mesure de température par contact. Pour cela, un embout peut être disposé à la place de l'ouverture 4. L'embout peut être adapté à une mesure buccale ou à une mesure par l'intermédiaire du conduit auditif externe. L'embout peut être à usage unique soit en étant jetable soit en étant interchangeable afin d'être stérilisé. Le capteur 5 est également adapté en conséquence, par des moyens connus de l'homme du métier.

La poignée 1 est d'une taille permettant la préhension à une ou deux mains. La poignée 1 peut être formée anatomiquement. Le revêtement de la poignée 1 peut être antidérapant. Selon une autre alternative, le revêtement de la poignée 1 peut comporter des propriétés antibactériennes.

Le corps 2 du boîtier comprend notamment une face 6 dirigée vers l'utilisateur et permettant l'interaction entre l'utilisateur et le système de mesure. La face opposée à la face 6 dirigée vers l'utilisateur peut être également formée de façon anatomique de sorte que le corps 2 du boîtier puisse reposer dans la main de l'utilisateur.

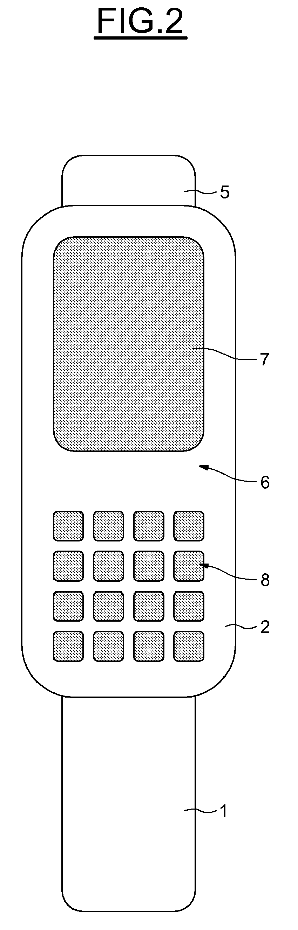

La face 6 du corps 2 du boîtier dirigée vers l'utilisateur est illustrée sur la figure 2. Le boîtier comprend un dispositif d'affichage 7 et un ensemble de touches 8, pouvant former un clavier. Des touches 8 peuvent être également disposées sur la périphérie du dispositif d'affichage 7.

Le dispositif d'affichage 7 peut être de type LCD, LCD rétroéclairé, LED ou utiliser toute autre technologie d'affichage portable.

Le dispositif d'affichage 7 peut s'étendre sur tout ou partie de la face 6 du corps 2 du boîtier dirigée vers l'utilisateur. Le dispositif d'affichage 7 et les touches peuvent être regroupées au sein d'un dispositif d'affichage 7 tactile.

Le corps 2 du boîtier peut également comprendre la source d'énergie 9 du système, et des moyens pour échanger la source d'énergie 9 épuisée par une source d'énergie neuve, ou pour recharger la source d'énergie, ou une combinaison des deux.

Le corps 2 du boîtier comprend également tout ou partie de l'électronique 10 apte à traiter les signaux reçus du capteur 5 et les signaux reçus des touches 8 et à émettre les signaux à destination du dispositif d'affichage 7. Cette électronique 10 comprend notamment une cartographie 11 et un moyen de détermination 12 d'au moins une action à entreprendre.

La cartographie 11 permet d'associer à des températures mesurées des actions à entreprendre. Par action à entreprendre, on entend une action que l'utilisateur du système de mesure doit entreprendre suite à la mesure de la température. Les actions à entreprendre peuvent comprendre appeler des services de secours médical, appeler un professionnel de santé, surveiller un état incertain, refroidir ou réchauffer le sujet dont la température a été mesurée, procéder à l'administration d'un traitement, en automédication ou éventuellement prescrit par un professionnel de santé, ou une combinaison d'une ou plusieurs de ces possibilités.

Selon un mode de réalisation, la cartographie 11 peut également être plus précise et comprendre une collection d'associations de températures à des actions à entreprendre, chaque association étant établie pour un ensemble de paramètres physiques du sujet. Les paramètres physiques du sujet peuvent comprendre l'âge, le poids, le sexe et l'espèce.

Selon un autre mode de réalisation, plusieurs sujets peuvent être suivis grâce au même système de mesure, l'ensemble des paramètres physiques de chaque sujet étant stocké dans des mémoires 13 ou des espaces mémoires distincts.

La cartographie 11 ou les mémoires 13 peuvent comprendre une partie modifiable par l'utilisateur, et dans lequel l'utilisateur peut introduire la fréquence de prise d'un médicament, ou des conditions de prise d'un médicament (heure de prise et, éventuellement, dose prise). Le système de mesure peut comprendre une confirmation des données saisies par l'utilisateur. Lorsque les conditions de prise d'un médicament sont satisfaites, le système de mesure le signale à l'utilisateur.

Il est également possible de tenir compte des paramètres physiques de l'environnement lors de la détermination des actions à entreprendre. Les paramètres physiques de l'environnement peuvent comprendre le taux d'humidité, la température de l'air, la date et l'heure courant ainsi que le rythme circadien courant. Par valeurs courantes, on entend les valeurs relatives au lieu d'utilisation du système de mesure, ces valeurs changeant lorsque l'utilisateur transporte son système de mesure.

La détermination des actions à entreprendre peut également prendre en compte les actions entreprises par l'utilisateur. Par exemple, l'utilisateur peut valider les actions réalisées parmi les actions à entreprendre précédemment déterminées et proposées par le système de mesure.

Dans les modes de réalisation alternatifs comprenant l'utilisation de paramètres physiques du sujet et/ou de paramètres physiques de l'environnement et/ou des actions entreprises, le moyen de détermination des actions à entreprendre fournit à la cartographie 11 les différents paramètres ou actions requis. Les paramètres physiques du sujet et/ou de paramètres physiques de l'environnement et/ou des actions entreprises peuvent être saisis par l'utilisateur et/ou déterminés par des capteurs correspondants.

Dans son fonctionnement, le système de mesure peut être relié à un serveur distant permettant le stockage des paramètres physiques du sujet et/ou de paramètres physiques de l'environnement et/ou des actions entreprises ainsi que le stockage de la partie modifiable de la cartographie 11 contenant la fréquence de prise d'un médicament, ou les conditions de prise d'un médicament. Les données stockées sur un serveur distant peuvent être supervisées ou modifiées par un professionnel de santé.

Le système de mesure peut comprendre également un dispositif d'affichage 7 déporté soit localement, soit sur un site distant. Le dispositif d'affichage 7 déporté peut être redondant par rapport au dispositif d'affichage 7 intégré au système de mesure ou le remplacer. Un tel système permet par exemple la prise de température de nombreux sujets et l'exploitation des résultats après les prises de températures, dans un lieu différent. Dans le cas où le dispositif d'affichage 7 déporté remplace le dispositif d'affichage 7 compris dans le système de mesure, un signal sonore ou lumineux à deux ou trois états peut être intégré au système de mesure de sorte que l'individu réalisant la mesure de température soit informé immédiatement d'une situation anormale ou dangereuse.

Le système de mesure peut être appliqué à un être humain ou à un être vivant, par exemple des animaux vertébrés ou des animaux homéothermes.

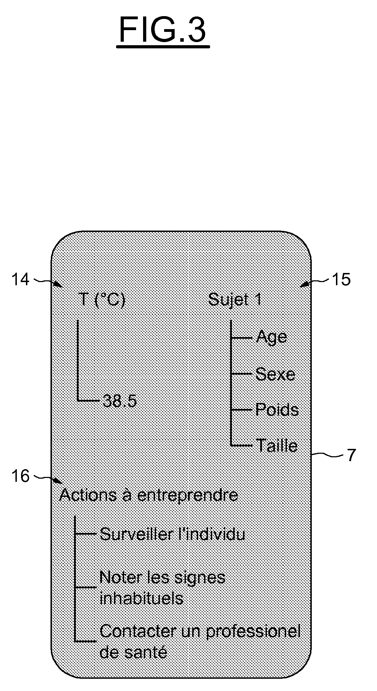

La figure 3 illustre un exemple d'informations représentées sur un dispositif d'affichage 7 permettant de mettre simultanément à la disposition de l'utilisateur des informations concernant la température mesurée 14 du sujet, les paramètres physiques 15 du sujet courant et les actions à entreprendre 16. D'autres informations peuvent être affichées, comme par exemple tout ou partie des paramètres physiques du sujet, des paramètres physiques de l'environnement et des actions entreprises. Un affichage 7 cyclique préprogrammé ou commandé par l'utilisateur peut permettre d'afficher plus de paramètres que de champs d'affichage 7 disponibles.

Dans un autre mode de réalisation, le système de mesure décrit ci-dessus est associé à un dispositif portable. Le système de mesure et le dispositif portable forment alors un dispositif de mesure.

Par dispositif portable, on entend un ordinateur portable, un téléphone intelligent (« smartphone » en langue anglaise), ou tout autre dispositif communicant apte à exécuter un logiciel. Le logiciel peut recevoir les informations du système de mesure par l'intermédiaire d'une liaison de données, filaire ou sans fil, notamment de type USB, Bluetooth, ou Wifi. Le logiciel peut accéder à la mémoire du dispositif portable afin d'y stocker des données temporaires nécessaires à l'exécution du logiciel ou des données persistantes comprenant l'indentification d'une ou des personnes suivies, ainsi qu'un historique des mesures précédemment effectuées. Alternativement, les données persistantes peuvent être hébergées sur un serveur distant et, à la demande, être téléchargées en vue d'être modifiées sur le dispositif portable ou directement modifiées sur le serveur.

Au sein du dispositif de mesure, le système de mesure peut interagir avec le dispositif portable afin de créer un historique des prises de température et afin de fournir des conseils, analyses et recommandations en fonction dudit historique et de la température courante.

Pour cela, le dispositif portable reçoit du système de mesure la mesure de température réalisée ainsi que les paramètres physiques du sujet associés. Alternativement, les paramètres physiques du sujet peuvent être saisis directement sur le dispositif portable. Si un historique de mesures de température est disponible dans le système de mesure, celui-ci est transmis en même temps ou après la transmission de la mesure de température et des paramètres physiques du sujet associés. Si des informations concernant la prescription et la prise d'un médicament sont fournies au système de mesure, elles peuvent être également transmises. Alternativement, les informations concernant la prescription et la prise d'un médicament peuvent être saisies directement sur le dispositif portable. Le dispositif portable comprend alors des moyens logiciels aptes à déterminer des actions à entreprendre en fonction de la mesure de température, de l'historique de mesures et des paramètres physiques du sujet. Le dispositif portable peut également comprendre une connexion de données avec un réseau, par exemple l'Internet, permettant de transmettre la mesure de température, de l'historique de mesures et des paramètres physiques du sujet à un spécialiste, et de recevoir en retour des conseils et prescriptions.

Dans un autre mode de réalisation, le dispositif d'affichage 7, la cartographie 11, les mémoires 13 et le moyen de détermination 12 d'au moins une action à entreprendre peuvent être déportés dans un dispositif portable, sous une forme matérielle ou logicielle. Le système de mesure comprend alors le capteur 5, avec ou sans contact, la source d'énergie 9 du système de mesure et des moyens de communication avec le dispositif portable.

Le moyen de détermination 12 peut alors être compris dans un logiciel exécuté sur le dispositif portable. D'une façon similaire au premier mode de réalisation, le moyen de détermination 12 reçoit des mesures du capteur 5 et les associe à des actions à entreprendre par l'intermédiaire de la cartographie 11.

Le moyen de détermination 12 peut également être associé à une collection d'actions à entreprendre dépendant de paramètres physiques du sujet. Le moyen de détermination 12 peut également suivre différents sujets et/ou mémoriser la fréquence ou les conditions de prise d'un médicament. Lorsque les conditions de prise d'un médicament sont satisfaites, le moyen de détermination 12 le signale à l'utilisateur.

Il est également possible de tenir compte des paramètres physiques de l'environnement lors de la détermination des actions à entreprendre. Les paramètres physiques de l'environnement peuvent comprendre le taux d'humidité, la température de l'air, la date et l'heure courant ainsi que le rythme circadien courant. Par valeurs courantes, on entend les valeurs relatives au lieu d'utilisation du système de mesure, ces valeurs changeant lorsque l'utilisateur transporte son système de mesure.

La détermination des actions à entreprendre peut également prendre en compte les actions entreprises par l'utilisateur. Par exemple, l'utilisateur peut valider les actions réalisées parmi les actions à entreprendre précédemment déterminées et proposées par le système de mesure.

Le moyen de détermination 12 peut comprendre l'utilisation de paramètres physiques du sujet et/ou de paramètres physiques de l'environnement et/ou des actions entreprises. Le moyen de détermination des actions à entreprendre fournit à la cartographie 11 les différents paramètres ou actions requis. Les paramètres physiques du sujet et/ou de paramètres physiques de l'environnement et/ou des actions entreprises peuvent être saisis par l'utilisateur et/ou déterminés par des capteurs correspondants.

Le moyen de détermination 12 peut être relié à un serveur distant permettant le stockage des paramètres physiques du sujet et/ou de paramètres physiques de l'environnement et/ou des actions entreprises ainsi que le stockage de la partie modifiable de la cartographie 11 contenant la fréquence de prise d'un médicament, ou les conditions de prise d'un médicament. Les données stockées sur un serveur distant peuvent être supervisées ou modifiées par un professionnel de santé. Le moyen de détermination 12 peut interagir directement avec les données stockées sur le serveur distant ou n'y stocker qu'une copie de sauvegarde des données présentes en mémoire.

Le dispositif d'affichage 7 est alors déporté sur le dispositif portable. Toutefois, l'affichage sur le dispositif portable peut être une copie de l'affichage présent sur le système de mesure. Le dispositif d'affichage 7 peut également être déporté ou dupliqué sur un site distant. Un tel système permet par exemple la prise de température de nombreux sujets et l'exploitation des résultats après les prises de températures, dans un lieu différent. Dans le cas où le dispositif d'affichage 7 déporté remplace le dispositif d'affichage 7 compris dans le système de mesure, un signal sonore ou lumineux à deux ou trois états peut être intégré au système de mesure de sorte que l'individu réalisant la mesure de température soit informé immédiatement d'une situation anormale ou dangereuse.

Le logiciel fonctionnant sur le dispositif portable peut présenter un affichage similaire à celui illustré sur la figure 3.

Le système de mesure permet de guider un individu vers un comportement cohérent avec la mesure de température du sujet, et simultanément, de mémoriser un ensemble de variables, dont la température.

Le système de mesure constitue également une aide au diagnostic pour le professionnel de santé en rendant disponible un historique objectif de différentes variables. Il ne dépend donc plus de renseignements empreints de subjectivité, pouvant se révéler imprécis ou faux et pouvant mener à un diagnostic erroné.

## Revendications

1. Système de mesure de la température interne d'un sujet, comprenant un capteur (5) de mesure de température et un dispositif d'affichage (7) apte à afficher la température mesurée, **caractérisé par le fait qu'**il comprend en outre :
une cartographie (11) comprenant un ensemble de valeurs de température associée chacune à des informations relatives à une ou plusieurs actions à entreprendre,
un moyen de détermination (12) d'au moins une action à entreprendre en fonction des informations contenues dans la cartographie (11) et de la température mesurée, et
au moins une mémoire (13) apte à mémoriser des paramètres des actions entreprises en rapport avec le sujet dont la température est mesurée.

2. Système de mesure selon la revendication 1, comprenant au moins une mémoire (13), apte à mémoriser des paramètres physiques d'au moins un sujet dont la température est mesurée.

3. Système selon la revendication 2, dans lequel les paramètres physiques sont choisis parmi l'espèce, l'âge, le poids et le sexe du sujet.

4. Système de mesure selon l'une quelconque des revendications précédentes, comprenant au moins une mémoire (13) apte à mémoriser des paramètres physiques de l'environnement du sujet dont la température est mesurée.

5. Système selon la revendication 4, dans lequel les paramètres physiques de l'environnement sont choisis parmi la température de l'air, l'hygrométrie, la date courante, l'heure courante et le rythme circadien.

6. Système selon l'une quelconque des revendications précédentes, dans lequel les paramètres des actions entreprises sont choisis parmi la date et l'heure d'au moins la dernière action entreprise.

7. Système selon l'une quelconque des revendications précédentes, dans lequel le capteur (5) de mesure de température est de type sans contact à distance.

8. Système selon l'une quelconque des revendications 1 à 6, dans lequel le capteur (5) de mesure de température est de type à contact.

9. Système selon la revendication 8, dans lequel le capteur (5) de mesure de température est apte à déterminer la température d'un sujet par mesure au niveau du conduit auditif externe.

10. Système selon la revendication 8, dans lequel le capteur (5) de mesure de température est apte à déterminer la température d'un sujet par mesure en contact au niveau de l'orifice buccal.

11. Système selon l'une quelconque des revendications précédentes, dans lequel le dispositif d'affichage (7) est déporté et relié par un moyen de communication à distance.

12. Système selon l'une quelconque des revendications précédentes, dans lequel le dispositif d'affichage (7) est apte à afficher simultanément la température mesurée (14) du sujet, au moins une action à entreprendre (16) et au moins un des paramètres choisi parmi les paramètres physiques de l'environnement, les paramètres physiques d'au moins un sujet (15) et/ou les paramètres de prises d'un traitement.

13. Système selon l'une quelconque des revendications précédentes, dans lequel le dispositif d'affichage (7), la cartographie (11) et le moyen de détermination (12) sont déportés de façon logicielle sur un dispositif portable.

14. Dispositif selon l'une quelconque des revendications 1 à 13, comprenant un dispositif portable apte à échanger des données avec le système de mesure, et à déterminer des conseils et prescriptions en fonction des données échangées avec le système de mesure.
